**Europäisches Patentamt**

**European Patent Office**

**Office européen des brevets**

(19)

(11) Numéro de publication : **0 171 300 B2**

(12)      # NOUVEAU FASCICULE DE BREVET EUROPEEN

(45) Date de publication du nouveau fascicule du brevet : **23.12.92 Bulletin 92/52**

(51) Int. Cl.⁵ : **G01N 33/28**

(21) Numéro de dépôt : **85401130.1**

(22) Date de dépôt : **07.06.85**

---

(54) **Dispositif pour la détermination du pointe de trouble d'un gazole.**

---

(30) Priorité : **12.06.84 FR 8409127**

(43) Date de publication de la demande :
**12.02.86 Bulletin 86/07**

(45) Mention de la délivrance du brevet :
**06.09.89 Bulletin 89/36**

(45) Mention de la décision concernant l'opposition :
**23.12.92 Bulletin 92/52**

(84) Etats contractants désignés :
**DE GB NL**

(56) Documents cités :
**EP-A- 0 041 458**
**WO-A-83/04098**
**FR-A- 1 357 123**
**FR-A- 1 417 250**
**US-A- 3 161 039**
**US-A- 3 187 557**
**US-A- 3 248 928**
**US-A- 3 713 743**

(73) Titulaire : **ELF FRANCE**
**137, Rue de l'Université**
**F-75007 Paris (FR)**

(72) Inventeur : **Laurent, Dominique**
**22 Avenue des Frères lumière**
**F-69008 Lyon (FR)**
Inventeur : **Fortunato, Gérard**
**49 Boulevard des Brotteaux**
**F-69006 Lyon (FR)**
Inventeur : **Damin, Bernard**
**1562 Grande Rue**
**F-69600 Oullins (FR)**
Inventeur : **Napoleon, Marcel**
**F-38390 St. Verges Mepieu Mont.Vercieu (FR)**

(74) Mandataire : **Hirsch, Marc-Roger**
**Cabinet Hirsch 34 rue de Bassano**
**F-75008 Paris (FR)**

EP 0 171 300 B2

Jouve, 18, rue Saint-Denis, 75001 PARIS

## Description

La présente invention se rapporte à un dispositif de détermination du point de trouble d'un gazole et elle résulte en partie des travaux effectués à l'Institut d'Optique Théorique et Appliquée par Monsieur Jacques VULMIERE.

Dans le dispositif visé par l'invention, on fait pénétrer de la lumière dans ledit échantillon et l'on utilise, pour déterminer le point de trouble, la diffusion de cette lumière qui se produit lorsque des cristaux paraffiniques prennent naissance dans l'échantillon, dont on abaisse à cet effet, tout en la mesurant, la température à partir d'une valeur initiale supérieure au point de trouble.

On a déjà proposé divers procédés et dispositifs pour cette détermination du point de trouble des gazoles, c'est-à-dire de la température à partir et en-dessous de laquelle apparaissent des cristaux au sein du gazole, lors de son refroidissement.

Ces procédés reposent essentiellement sur le rapide changement des propriétés optiques du gazole liquide au point de trouble, et font appel soit à un phénomène de dépolarisation d'un faisceau lumineux initialement polarisé, après qu'il ait traversé un échantillon de liquide dans lequel apparaissent les cristaux, soit à une diffusion d'un faisceau lumineux dans un échantillon lors de la formation des cristaux, la température de l'échantillon étant, dans tous les cas, contrôlée et progressivement abaissée jusqu'à ce que se manifeste le changement de propriétés optiques.

Un procédé et un dispositif connus (EP-A-0041458) utilisent une cellule à échantillon traversée par un flux lumineux dans une direction donnée sans que celui-ci agisse sur le détecteur photoélectrique associé à la cellule, tant que l'échantillon examiné est exempt de cristaux. Lorsque ceux-ci se forment (point de trouble), la lumière est diffusée vers le détecteur qui est à cet effet orientée dans une direction perpendiculaire à la direction précitée. Dans le dispositif connu, il y a risque d'émission de lumière parasite vers le détecteur, même en l'absence de cristaux, par exemple par des effets de réflexion sur les parois de la cellule.

Un autre procédé connu (FR-A-1417250) est basé sur la polarisation et la dépolarisation de la lumière, et non sur la diffusion, qui est le phénomène utilisé dans le dispositif selon la présente invention.

Dans un dispositif connu et disponible sur le marché sous le nom de Cloud-Point-Analyser, est mis en oeuvre le procédé consistant à homogénéiser l'échantillon de la cellule de test, à mettre en marche une barrière photo-électrique dont le rayon lumineux traverse la cellule, puis à faire baisser la température de la cellule test. Dans ce procédé, qui est différent de celui mis en oeuvre dans l'invention, il est nécessaire de calibrer la cellule photoélectrique après chaque nouvelle homogénéisation d'un nouvel échantillon.

La présente invention propose un dispositif permettant une détermination rapide et précise du point de trouble.

Ce dispositif présente en outre l'avantage d'être simple et fiable, et de fournir des résultats offrant une bonne corrélation avec la méthode visuelle utilisée habituellement.

Le dispositif pour la détermination du point de trouble d'un gazole, notamment pour usage dans une installation de mélange pour l'obtention d'un gazole répondant à des spécifications déterminées, comportant des moyens optiques de détection du point de formation de cristaux paraffiniques dans un échantillon dudit gazole contenu dans une cellule ayant une chambre de mesure équipée d'une sonde de température et d'une entrée et une sortie pour ledit gazole, ladite cellule étant disposée sur un chemin optique entre une source de lumière et un détecteur photoélectrique propre à délivrer un courant électrique sur l'action d'un flux lumineux, et comportant des moyens à base d'éléments à effet Peltier pour réduire progressivement la température de cet échantillon, est caractérisé en ce que lesdit moyens optiques comprennent, disposés successivement sur un axe optique rectilinéaire commun:

- ladite source de lumière,
- un diaphragme comportant un trou central,
- un moyen de filtre combinant des propriétés anticaloriques propres à arrêter le rayonnement infrarouge émis par ladite source et des propriétés de filtration spectrale,
- un objectif,
- ladite cellule disposée perpendiculairement audit axe optique et comportant dans un corps une chambre intérieure de mesure dans laquelle débouchent des conduits d'amenée et d'évacuation du gazole et qui comprend deux fenêtres transparentes formées par les lames parallèles pour le passage du faisceau focalisé par ledit objectif, et une gorge annulaire formant collecteur reliée à une source d'air comprimé et dans laquelle débouchent des passages susceptibles de créer des jets d'air sur la surface externe desdites fenêtres pour leur désembuage,
- un écran piège à lumière,
- ledit détecteur photoélectrique disposé coaxialement par rapport audit écran piège à lumière, ce détecteur étant nettement plus large que ledit écran,

ledit objectif étant adapté pour que l'image dudit trou, après passage dans ladite chambre comportant un échantillon de gazole ne manifestant aucune trouble, soit focalisé sur ledit écran piège à lumière et que ledit détecteur, ne recevant aucune lumière, débite un courant nul ou sensiblement nul, la lumière diffusée par l'échantillon, dès l'apparence de cristaux paraffini-

ques dans ledit échantillon de gazole, étant recueillie sur le détecteur produisant ainsi une variation brusque et importante, par rapport à ladite valeur nulle ou sensiblement nulle, du niveau de courant debité, en ce qu'il comprend des moyens de réglage de la température de l'échantillon associés à ladite cellule, ces moyens comprenant au moins une sonde thermique pour la mesure de la température du corps de la cellule, et en ce que le courant délivré par ledit détecteur à l'apparition d'un trouble dans le gazole de ladite chambre est utilisé pour commander le cycle thermique desdits éléments à effet Peltier.

Les caractèristiques et avantages de l'invention ressortiront mieux de la description suivante, donnée uniquement à titre d'exemple, en référence aux dessins annexés, dans lesquels:

- la figure 1 illustre schématiquement le dispositif permettant la mise en oeuvre du procédé selon l'invention;
- les figures 2 et 3 sont deux vues d'une cellule équipant le dispositif selon l'invention, respectivement en coupes transversale et axiale;
- la figure 4 est une vue partielle, à plus grande échelle, de la figure 3;
- la figure 5 est un schéma illustrant la variation du courant délivré par le détecteur du dispositif en fonction de la température du gazole dans la cellule.

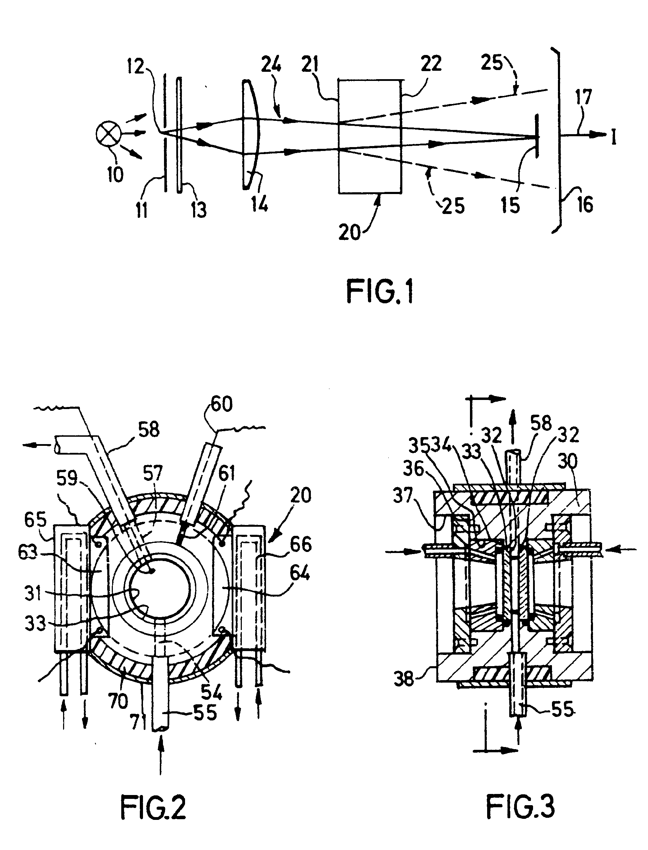

Le dispositif selon l'invention est schématisé à la figure 1. Il comprend une source lumineuse 10, par exemple une lampe quartz à méthode, et, successivement, alignés sur un même axe, un diaphragme 11 comportant un trou central 12 sur ledit axe, ce trou 12 ayant un diamètre réduit, par exemple 2 mm, un filtre interférentiel 13, un objectif 14 formant l'image du trou 12 sur un écran 15, opaque, constituant piège à lumière, et, derrière cet écran, un détecteur photoélectrique 16, de grand diamètre, propre à délivrer un courant I, en 17, quand il reçoit un flux lumineux.

Une cellule 20, contenant le gazole à analyser, est disposée entre l'objectif 14 et l'écran 15; cette cellule dont la structure est donnée plus en détail ciaprès comporte sur le trajet du faisceau lumineux 24 issu de l'objectif 14, deux fenêtres à faces parallèles 21 et 22 ne déviant pas ce faisceau.

Lorsque le gazole contenu dans la cellule 20 ne comporte pas de cristaux, toute la lumière issue de la source 10, plus précisément du trou 12, est arrêtée par l'écran opaque 15. Le détecteur 16, qui ne reçoit aucune lumière, débite en 17 un courant nul ou sensiblement nul.

Dès l'apparition de cristaux au sein du gazole, la lumière est diffusée au niveau de la cellule (rayons 25) et tombe sur la partie sensible du détecteur 16, non masquée par l'écran 15, ce détecteur délivrant alors un courant I, ce dernier croissant très rapidement en fonction de l'abaissement de la température.

Cette croissance rapide permet de mesurer avec une précision suffisante, de l'ordre du ½°C, la température d'apparition des cristaux dans le gazole. Le même courant est avantageusement utilisé pour contrôler le refroidissement de la cellule 20, comme on va le voir ci-après.

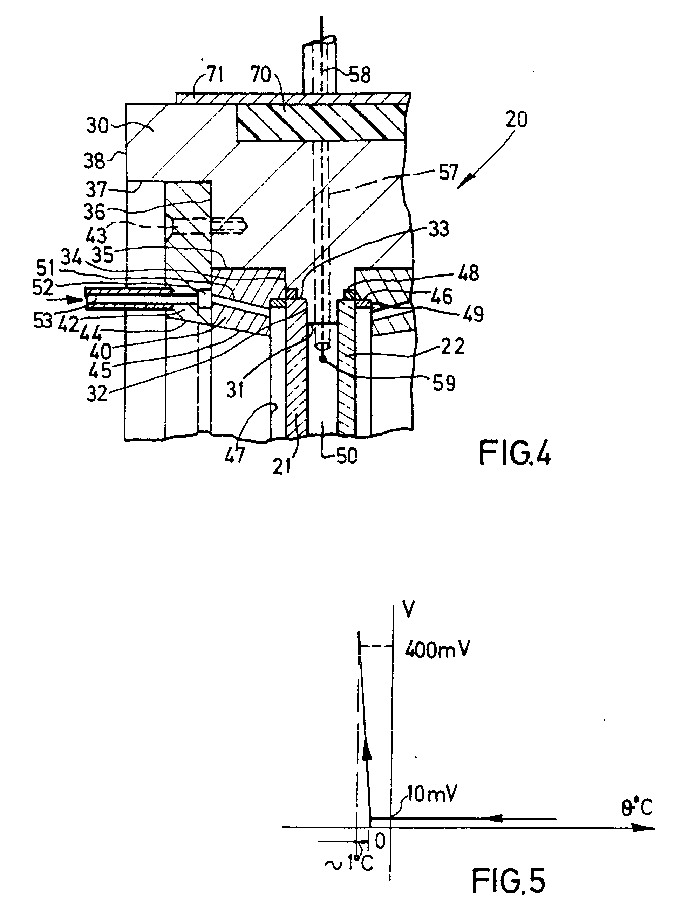

La cellule 20, dans la forme de réalisation représentée aux figures 2 et 3 et, partiellement, à plus grande échelle à la figure 4, consiste en un corps métallique 30 de forme générale cylindrique, selon l'axe duquel est pratiqué un passage central. Ce passage central qui traverse le corps 30 de part en part est symétique par rapport au plan de symétrie de ce corps, perpendiculaire à l'axe de celui-ci. Il comporte, à partir d'un alésage central 31, de part et d'autre de celuici, une succession, de portées annulaires concentriques, 32, 34, 36, 38 qui relient entre elles des alésages de diamètre croissant, 33, 35, 37. La portée 38 constitue la face extérieure du corps 30. La figure 4 illustre en détail cette disposition pour la partie gauche du corps, vu en coupe axiale, et qui se reproduit symétriquement pour la partie droite.

Les portées les plus intérieures, 32, reçoivent les lames circulaires transparentes 21, 22, à faces parallèles, qui constituent les fenêtres de la cellule 20, et ont un diamètre légèrement inférieur à celui de l'alésage 33. Chaque lame est maintenue en place par une bague 40, dont le diamètre correspond à celui de l'alésage 35, et qui est logé dans ce dernier, dont la profondeur correspond à épaisseur de la bague 40. A son tour, chaque bague 40 est retenue par une rondelle 42, d'un diamètre correspondant à celui de l'alésage 37 et logée dans ce dernier, la face inférieure de cette rondelle 42 prenant appui sur la face extérieure de la bague 40. Des moyens sont prévus pour plaquer la rondelle 42 contre la portée 26, par exemple des vis 43.

Les alésages intérieurs de la rondelle 42 et de la bague 40, 44, 45 respectivement, forment un passage qui laisse dégagée la majeure partie de chaque lame transparente 21, 22.

Un décrochement annulaire 46 est pratiqué dans la face intérieure de la bague 40, en regard de la lame adjacente, et ménage sur cette face une portée 47, espacée de ladite lame.

Des joints d'étanchéité 48, 49 sont disposés autour des lames 21, 22, et entre celles-ci et les bagues 40, pour ménager entre ces lames une chambre étanche 50.

Dans chaque bague 40, sont ménagés une série de passages 51, traversant ladite bague et débouchant par une extrémité sur la portée 47, tandis que dans la face intérieure de la rondelle 42 est ménagée une gorge annulaire 52, formant collecteur, dans laquelle débouchent les autres extrémités des passages 51. Un conduit 53, relié à une source d'air non représentée, traverse la rondelle 42 et débouche dans la gorge 52. Par l'envoi d'air sous pression par le conduit 53, on provoque la création de jets d'air sur les faces extérieures des lames 21, 22, et leur désembuage éventuel.

Un canal 54 raccordé par une canalisation 55 d'amenée du gazole à analyser est ménagé dans l'épaisseur du corps 30 et débouche dans la chambre 50 et un canal 57 ménagé également dans le corps 30 et débouchant dans la chambre 50, sensiblement à l'opposé du précédent, est relié à une canalisation 58 d'évacuation du gazole.

Une sonde thermoélectrique 59 est disposée dans le canal 57, son élément sensible étant placé dans la chambre 50, pour la mesure de la température du gazole dans ladite chambre.

Une autre sonde 60, logée dans un trou borgne 61 pratiqué dans le corps 30, permet la mesure de la température de ce corps.

Dans des logements symétriques du corps 30, proches de la chambre 50, sont disposés deux éléments Peltier 63, 64, à chacun desquels est associé un radiateur 65, 66 respectivement, à circulation intérieure d'eau, pour l'évacuation des calories ou frigories prenant naissance dans lesdits éléments.

De plus, pour un meilleur calorifugeage de la cellule, une couche isolante 70, par exemple en mousse de polystyrène, est injectée entre la paroi de la cellule et un revêtement protecteur 71.

Le courant 17 délivré par le détecteur 16 à l'apparition d'un trouble dans le gazole de la chambre 50 est utilisé pour commander le cycle thermique des éléments à effet Peltier 63, 64 refroidissant la cellule 20, ainsi que pour commander le cycle d'évacuation de la cellule par le gazoie une fois la mesure effectuée.

Le volume de la chambre 50 est très réduit, ce qui permet une variation rapide de la température du gazole qu'elle contient et des temps de mesure très courts.

Par ailleurs, la constitution de la cellule permet d'opérer avec un système optique propre et qui reste propre. La variation de courant obtenue entre l'absence de cristaux et la première apparition de ceux-ci est très voisine d'une variation par tout ou rien puisque la tension d'obscurité (absence de cristaux et lumière piégée par l'écran 15) est par exemple de 10 mV, tandis que lors de l'apparition de ceux-ci et pour une variation de température inférieure à 1°C, cette tension monte à 400 mV par exemple (figure 5).

Pour un meilleur rendement encore, au lieu de constituer le piège à lumière 15 par un écran noir mat, on peut utiliser un piège à lumière de Vulmière (cône réfléchissant dont la pointe est tournée vers la source) ou un miroir sphérique centré sur la chambre 50. De même, le filtre interférentiel 13 peut être remplacé par un filtre anticalorique (destiné à arrêter le rayonnement infrarouge perturbant les mesures) ou un filtre coloré pour une meilleure exploitation de la sensibilité du détecteur. Un filtre interférentiel peut d'ailleurs combiner ces deux effets.

Il est à noter que le dispositif selon l'invention assure une très bonne corrélation avec la méthode visuelle utilisée habituellement, beaucoup moins précise et d'une mise en oeuvre longue.

Le dispositif selon l'invention se prête ainsi particulièrement bien au pilotage d'une installation de mélange de gazoles, destinée à l'obtention d'un gazole répondant à des spécifications déterminées.

Bien entendu, la présente invention n'est pas limitée aux modes de réalisation décrits et représentés et elle est susceptible de nombreuses variantes accessibles à l'homme de l'art sans que l'on ne s'écarte de l'étendue de la protection des revendications.

## Revendications

1. Dispositif pour la détermination du point de trouble d'un gazole, notamment pour usage dans une installation de mélange pour l'obtention d'un gazole répondant à des spécifications déterminées, comportant des moyens optiques de détection du point de formation de cristaux paraffiniques dans un échantillon dudit gazole contenu dans une cellule ayant une chambre de mesure équipée d'une sonde de température et d'une entrée et une sortie pour ledit gazole, ladite cellule étant disposée sur un chemin optique entre une source de lumière et un détecteur photoélectrique propre à délivrer un courant électrique sur l'action d'un flux lumineux, et comportant des moyens à base d'éléments à effet Peltier pour réduire progressivement la température de cet échantillon, caractérisé en ce que lesdit moyens optiques comprennent, disposés successivement sur un axe optique rectilinéaire commun:
   - ladite source de lumière (10),
   - un diaphragme (11) comportant un trou central (12),
   - un moyen de filtre (13) combinant des propriétés anticaloriques propres à arrêter le rayonnement infrarouge émis par ladite source (10) et des propriétés de filtration spectrale,
   - un objectif (14),
   - ladite cellule (20) disposée perpendiculairement audit axe optique et comportant dans un corps (30) une chambre intérieure de mesure (50) dans laquelle débouchent des conduits (55, 58) d'amenée et d'évacuation du gazole et qui comprend deux fenêtres transparentes formées par les lames parallèles (21, 22) pour le passage du faisceau focalisé par ledit objectif, et une gorge annulaire (52) formant collecteur reliée à une source d'air comprimé et dans laquelle débouchent des passages (51) susceptibles de créer des jets d'air sur la surface externe desdites fenêtres pour leur dé- sembuage,
- un écran piège à lumière (15),
- ledit détecteur photoélectrique (16) disposé coaxialement par rapport audit écran piège à lumière, ce détecteur (16) étant nettement plus large que ledit écran,

ledit objectif étant adapté pour que l'image dudit trou, après passage dans ladite chambre (50) comportant un échantillon de gazole ne manifestant aucune trouble, soit focalisée sur ledit écran piège à lumière (15) ait que ledit détecteur (16), ne recevant aucune lumière, débitai un courant nul ou sensiblement nul, la lumière diffusée par l'échantillon, dès l'apparence de cristaux paraffiniques dans ledit échantillon de gazole, étant recueillie sur le détecteur (16) produisant ainsi une variation brusque et importante, par rapport à ladite valeur nulle ou sensiblement nulle, du niveau de courant débité, en ce qu'il comprend des moyens de réglage de la température de l'échantillon associés à ladite cellule, ces moyens comprenant au moins une sonde thermique (60) pour la mesure de la température du corps (30) de la cellule (20), et en ce que le courant délivré par ledit détecteur (16) à l'apparition d'un trouble dans le gazole de ladite chambre (50) est utilisé pour commander le cycle thermique desdits éléments (63, 64) à effet Peltier.

2. Dispositif selon la revendication 1, caractérisé en ce que le courant délivré par ledit détecteur (16) à l'apparition d'un trouble dans le gazole de ladite chambre (50) est utilisé pour commander le cycle d'évacuation de la cellule par le gazole une fois la mesure effectuée.

3. Dispositif selon l'une des revendications 1 ou 2, caractérisé en ce que l'écran piège à lumière est constitué par un écran noir mat (15).

4. Dispositif selon l'une des revendications 1 ou 2, caractérisé en ce que le piège à lumière est constitué par un cône réfléchissant dont la pointe est tournée vers la source ou par un miroir sphérique centré sur la chambre de mesure.

**Patentansprüche**

1. Vorrichtung zur Ermittlung des Trübungspunktes eines Dieselöls, insbesondere zur Benutzung in einer Mischanlage zur Erzeugung eines Dieselöls das bestimmten Spezifikationen entspricht welches optische Mittel zur Erfassung des Formationspunktes von paraffinischen Kristallen in einem Muster dieses Dieselöls aufweist, das in einer Zelle enthalten ist die eine mit einer Temperatursonde versehene Messkammer und einen Eintritt und einen Austritt für dieses Dieselöl aufweist, wobei diese Zelle. auf einem optischen Weg zwischen einer Lichtguelle und einem fotoelektrischen Sensor angebracht ist, der geeignet ist, elektrischen Strom auf eine Lichtstromleistung abzugeben, und auf Peltier-Elementen basierte Mittel aufweist, um die Temperatur dieses Muster: nach und nach zu reduzieren, dadurch gekennzeichnet, dass die nacheinander auf einer optischen geradlinigen gemeinsamen Achse liegenden optischen Mittel folgendes enthalten:
- die Lichtquelle (10),
- eine Blende (11) mit einem zentralen Loch (12),
- ein Filter (13) der antikalorische Figenschaften zum Aufhalten der von der Quelle (10) abgegebenen Infrarotstrahlen und spektralfiltrationseigenschaften aufweist,
- ein Objektiv (14),
- die senkrecht zu der optischen Achse liegende Messzelle (20) die in einem Körper (30) eine innere Messkammer (50) aufweist, in der Dieselöleinlass- und Auslassleitungen (55, 58) münden und die zwei durch parallele Lamellen (21, 22) gebildete durchsichtige Fenster für den Durchlass der von dem Objektiv gebündelten Strahlen aufweist und einen ringförmigen Durchlass (52) der einen mit einer Pressluftquelle verbundenen Kollektor bildet und in den Durchlässe (51) münden, die über Mittel zum Richten von Luftstrahlen auf das Äussere der Fenster verfügen um diese von dem Feuchtigkeitsbeschlag zu befreien,
- eine Lichtfalle (15),
- den koaxial zu der Lichtfalle angebrachten elektrischen Sensor (16), wobei dieser Sensor (16) wesentlich breiter ist als die Lichtfalle, wobei das Objektiv derart angepasst ist, dass das Bild des Lochs, nach Durchgang durch die ein Dieselölmuster ohne jegliche Trübung enthaltenden Kammer (50) auf die Lichtfalle (15) fokussiert wird und der Sensor (16), der keinerlei Licht erhält, keinen oder fast keinen Strom abgibt, wobei das von dem Muster diffundierte Licht sofort bei Erscheinen von paraffinischen Kristallen in dem Dieselölmuster auf den Sensor (16) gesammelt wird und so eine heftige und erhebliche Schwankung des abgegebenen Stromniveaus hervorruft, im Verhältnis zu dem Nullwert oder Fast-Nullwert,

und dass sie über Mittel zur Regulierung der Temperatur des Musters in Verbindung mit der Zelle verfügt, wobei diese Mittel wenigstens einen Temperaturfühler (60) zum Messen der Temperatur des Körpers (30) der Zelle (20) aufweisen, und dass der von dem Sensor (16) abgegebene Strom bei Erscheinen einer Trübung im Dieselöl der Kammer (50) benutzt wird, um den Betriebszyklus der Peltier-Elemente (63, 64) zu steuern.

2. Vorrichtung nach Anspruch 1, dadurch gekennzeichnet, dass der von dem Sensor (16) abgegebene Strom bei Erscheinen einer Trübung im Dieselöl der Kammer (50) benutzt wird, um den Entleerungszyklus der Zelle durch das Dieselöl nach dem Messen zu steuern.

3. Vorrichtung nach einem der Ansprüche 1 oder 2, dadurch gekennzeichnet, dass die Lichtfalle aus einem schwarzen, matten Schirm (15) besteht.

4. Vorrichtung nach einem der Ansprüche 1 oder 2, dadurch gekennzeichnet, dass diese Lichtfalle als ein mit seiner Spitze der Lichtquelle zugekehrter reflektierender Kegel ausgebildet ist, oder aus einem auf die Messkammer zentrisch ausgerichteten sphärischen Spiegel besteht.

**Claims**

1. Device for determining the cloud point of a gas oil, notably for use in a blending installation for obtaining a gas oil that meets determined specifications, comprising optical means for detecting the point of formation of paraffin-type crystals in a sample of said gas oil contained in a cell provided with a measurement chamber fitted with a temperature sensor and an inlet and outlet for said gas oil, said cell being arranged on an optical path between a light source and a photoelectric detector adapted to furnish an electrical current under the effect of luminous flux, and including Peltier effect-based means for progressively reducing the temperature of said sample, characterized in that said optical means comprise the following arranged successively on a common rectilinear optical axis:
   - said light source (10),
   - a diaphragm (11) provided with a central aperture (12),
   - filter means (13) combining heat-protective properties adapted to act as a barri.er to infrared radiation emitted by said source (10) and spectral filtering properties,
   - an objective lens (14),
   - said cell (20) being arranged perpendicular to said optical axis and including in a body (30) an internal measurement chamber (50) in which conduits (55, 58) for introducing and evacuating said gas oil terminate and which includes two transparent windows formed by thin parallel plates (21, 22) for the passage of a beam focussed by said objective lens, and an annular channel (52) forming a common duct linked to a source of compressed air and in which passages (51) terminate adapted to set up air jets on the outer surface of said windows for demisting thereof,
   - light trap means (15),
   - said photoelectric detector (16) being arranged coaxially with respect to said light trap means, and said detector (16) being distinctly wider than said light trap means,
said objective lens being adapted so that the image of said aperture, after passing through said chamber (50) containing a sample of gas oil exhibiting no clouding gets focussed on said light trap means (15) and said detector (16), receiving no light, output zero or substantially zero current, light diffused by said sample as soon as paraffin type crystals start to form in said gas oil sample being collected on said detector (16) thus producing a sharp and significant change in the current output from said detector compared to said zero or suhstantially zero value, and in that it includes means for regulating the temperature of the sample associated with said cell, said means comprising at least one heat sensor (60) for measuring the temperature of the body (30) of the cell (20), and in that the current output from said detector (16) upon clouding of said sample of gas oil contained in said chamber (50) appearing is employed for controlling the thermal cycle of said Peltier effect-based means (63, 64).

2. Device according to claim 1, characterized in that the current supplied by said detector (16) upon the appearance of clouding of the gas oil of said chamber (50) is used to control the cell's evacuation cycle by the gas oil after measurement.

3. Device according to one of claims 1 or 2, characterized in that the light trap is constituted by a mat black screen (15).

4. Device according to one of claims 1 or 2, characterized in that the light trap is constituted by a reflecting cone the point of which is directed towards the source or by a spherical mirror centered on the measurement chamber.

FIG.1

FIG.2

FIG.3

FIG.4

FIG.5